(19) Europäisches Patentamt
European Patent Office
Office européen des brevets

(11) Numéro de publication: **0 399 900 A1**

(12) **DEMANDE DE BREVET EUROPEEN**

(21) Numéro de dépôt: **90401367.9**

(51) Int. Cl.5: **C07D 233/93**

(22) Date de dépôt: **22.05.90**

(30) Priorité: **23.05.89 FR 8906702**

(43) Date de publication de la demande: **28.11.90 Bulletin 90/48**

(84) Etats contractants désignés: **AT BE CH DE DK ES FR GB GR IT LI LU NL SE**

(71) Demandeur: **RHONE-POULENC SANTE**
**20, avenue Raymond Aron**
**F-92160 Antony(FR)**

(72) Inventeur: **Massonneau, Viviane**
**Charrière Blanche, Le Tilleul**
**F-69130 Ecully(FR)**
Inventeur: **Mulhauser, Michel**
**Immeuble "Frênes 4" Résidence Charrière Blanche**
**F-69130 Ecully(FR)**

(74) Mandataire: **Pilard, Jacques et al**
**RHONE-POULENC SANTE, Service Brevets,**
**20 Avenue Raymond Aron**
**F-92165 Antony Cédex(FR)**

(54) **Procédé de préparation du (chloro-2 éthyl)-1 méthyl-2 nitro-5 imidazole.**

(57) Procédé de préparation du (chloro-2 éthyl)-1 méthyl-2 nitro-5 imidazole par action du sulfate de di- chloro-2 éthyle sur un dérivé de l'imidazole de formule générale :

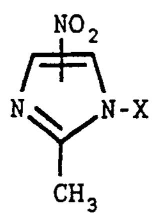

(X représente un atome d'hydrogène ou un radical éliminable par hydrolyse acide ou par alcoolyse), suivie de l'hydrolyse acide ou de l'alcoolyse du produit obtenu.

EP 0 399 900 A1

**PROCEDE DE PREPARATION DU (CHLORO-2 ETHYL)-1METHYL-2 NITRO-5 IMIDAZOLE**

La présente invention concerne un nouveau procédé de préparation du (chloro-2 éthyl)-1 méthyl-2 nitro-5 imidazole.

Le (chloro-2 éthyl)-1 méthyl-2 nitro-5 imidazole est un intermédiaire particulièrement intéressant pour la synthèse de l'(éthylsulfonyl-2 éthyl)-1 méthyl-2 nitro-5 imidazole (ou tinidazole) qui présente des propriétés thérapeutiques remarquables.

Il est connu de préparer un (halogéno-2 éthyl)-1 méthyl-2 nitro-5 imidazole par halogénation de l'-(hydroxy-2 éthyl)-1 méthyl-2 nitro-5 imidazole [Indian J. Chem. Sci., 44-46 (1982)] qui peut être obtenu par hydroxyéthylation d'un méthyl-2 nitro-4 (ou -5) imidazole.

Il a maintenant été trouvé, et c'est ce qui fait l'objet de la présente invention, que le (chloro-2 éthyl)-1 méthyl-2 nitro-5 imidazole peut être obtenu par action du sulfate de di-chloro-2 éthyle de formule :

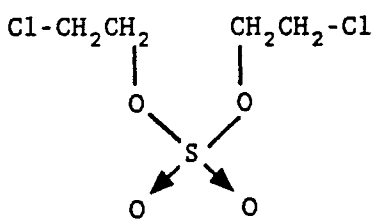

(I)

sur un dérivé de l'imidazole de formule générale :

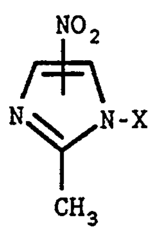

(II)

dans laquelle X représente un atome d'hydrogène ou un groupement facilement éliminable par hydrolyse acide ou par alcoolyse choisi parmi les radicaux hydroxyméthyle, alcoxyméthyle dont la partie alcoyle contient 1 à 4 atomes de carbone, acyloxyméthyle dont la partie acyle contient 1 à 4 atomes de carbone ou un radical éthylénique allylique, tel que le radical allyle, ou un radical arylméthyle, tel que le radical benzyle, suivie de l'hydrolyse acide ou de l'alcoolyse du produit de condensation.

La condensation du sulfate de formule (I) sur le dérivé de l'imidazole de formule générale (11) est effectuée à une température comprise entre 60 et 120 °C en présence éventuellement d'un solvant organique choisi parmi les esters tel que l'acétate de méthyle, l'acétate d'éthyle ou le diacétate de glycol, les cétones telles que la méthylisobutylcétone, les éthers tels que le méthyltertiobutyléther, les hydrocarbures aliphatiques ou aromatiques éventuellement halogénés tels que le benzène, le toluène, le xylène, le chloroforme, le dichlorométhane ou le chlorobenzène ou les nitriles tels que l'acétonitrile.

Le produit de condensation peut être solubilisé :

- soit dans une solution aqueuse d'un acide minéral fort tel que, par exemple, l'acide sulfurique ou l'acide chlorhydrique,
- soit dans un alcool, tel que par exemple, le méthanol ou l'éthanol.

Lorsque le produit de condensation est solubilisé dans l'eau acidifiée, le (chloro-2 éthyl)-1 méthyl-2 nitro-5 imidazole est extrait selon les techniques habituelles après alcalinisation du mélange réactionnel à un pH voisin de 10.

Lorsque le produit de condensation est solubilisé dans un alcool, le (chloro-2 éthyl)-1 méthyl-2 nitro-5 imidazole est isolé selon les techniques habituelles sans traitement préalable du mélange réactionnel.

Pour la mise en oeuvre du procédé, il n'est pas nécessaire d'isoler le produit de condensation intermédiaire, l'hydrolyse ou l'alcoolyse pouvant être enchaînées dans le même appareil.

Le sulfate de formule (I) peut être obtenu selon le procédé décrit par R. Levaillant, Ann. Chim. 11/6, 532 (1936).

Le dérivé de l'imidazole de formule générale (II) dans lequel X représente un groupement éliminable par hydrolyse ou alcoolyse peut être préparé dans les conditions décrites dans le brevet anglais GB 1 026 631.

Le (chloro-2 éthyl)-1 méthyl-2 nitro-5 imidazole peut être transformé en tinidazole dans les conditions décrites dans le brevet suisse CH 582 678 ou dans le brevet français FR 2 268 017.

L'exemple suivant, donné à titre non limitatif, illustre le procédé selon l'invention.

## EXEMPLE

Dans un ballon muni d'un agitateur, on introduit 44,6 g de sulfate de di-chloro-2 éthyle (0,2 mole) et 21 g d'acétoxyméthyl-1 méthyl-2 nitro-4 imidazole (0,104 mole) puis on chauffe à 120 °C pendant 4 heures. Après refroidissement à 80 °C, on ajoute 100 cm3 d'éthanol puis chauffe à 80 °C pendant 1 heure.

Après refroidissement à une température voisine de 20 °C et concentraticn, on obtient une huile (55,2 g), dont le dosage par chromatographie liquide à haute performance (CLHP) montre qu'elle contient 30,8 % de (chloro-2 éthyl)-1 méthyl-2 nitro-5 imidazole.

Le rendement en (chloro-2 éthyl)-1 méthyl-2 nitro-5 imidazole est de 86,8 % par rapport à l'acétoxyméthyl-1 méthyl-2 nitro-4 imidazole mis en oeuvre.

A l'huile obtenue, on ajoute 30 cm3 d'eau puis on extrait par 4 fois 100 cm3 de dichlorométhane. Après concentration de la phase organique, le produit obtenu est solubilisé dans l'éther isopropylique. On chauffe au reflux. Après décantation et refroidissement, le produit qui précipite est séparé par filtration. On obtient ainsi 8,93 g de (chloro-2 éthyl)-1 méthyl-2 nitro-5 imidazole.

Le rendement en (chloro-2 éthyl)-1 méthyl-2 nitro-5 imidazole est de 45,5 % par rapport à l'acétoxyméthyl-1 méthyl-2 nitro-4 imidazole mis en oeuvre.

## Revendications

1 - Procédé de préparation du (chloro-2 éthyl)-1 méthyl-2 nitro-5 imidazole caractérisé en ce que l'on fait réagir le sulfate de di-chloro-2 éthyle de formule :

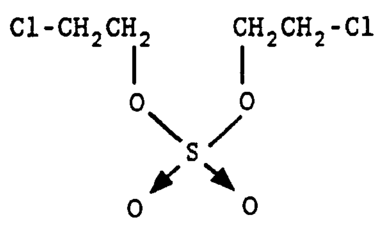

sur un dérivé de l'imidazole de formule générale :

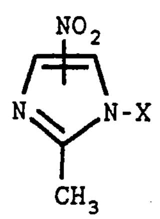

dans laquelle X représente un atome d'hydrogène ou un radical éliminable par hydrolyse acide ou alcoolyse, puis hydrolyse ou alcoolyse le produit obtenu et isole le (chloro-2 éthyl)-1 méthyl-2 nitro-5 imidazole.

2 - Procédé selon la revendication 1 caractérisé en ce que le radical éliminable par hydrolyse acide ou alcoolyse est choisi parmi les radicaux hydroxyméthyle, alcoxyméthyle dont la partie alcoyle contient 1 à 4 atomes de carbone, acyloxyméthyle dont la partie acyle contient 1 à 4 atomes de carbone ou un radical éthylénique allylique ou un radical arylméthyle.

3 - Procédé selon l'une des revendications 1 ou 2 caractérisé en ce que l'on opère dans un solvant organique choisi parmi les esters, les éthers, les cétones, les hydrocarbures aliphatiques ou aromatiques éventuellement halogénés et les nitriles.

4 - Procédé selon la revendication 3 caractérisé en ce que le solvant est choisi parmi l'acétate de méthyle, l'acétate d'éthyle, le diacétate de glycol, le méthyltertiobutyléther, la méthylisobutylcétone, le chloroforme, le dichlorométhane, le benzène, le toluène, le xylène, le chlorobenzène et l'acétonitrile.

5 - Procédé selon la revendication 1 caractérisé en ce que l'hydrolyse du produit de condensation est effectuée en présence d'un acide fort choisi parmi l'acide sulfurique et l'acide chlorhydrique.

6 - Procédé selon la revendication 1 caractérisé en ce que l'alcoolyse est effectuée par chauffage en présence d'un alcool choisi parmi le méthanol et l'éthanol.

Office européen des brevets

# RAPPORT DE RECHERCHE EUROPEENNE

Numero de la demande

EP 90 40 1367

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | BE-A- 698 067 (KRKA TOVARNA YDRAVIL)<br>--- | | C 07 D 233/93 |
| D,A | CH-A- 582 678 (CRC COMPAGNIA DI RICERCA CHIMICA S.A.)<br>--- | | |
| D,A | FR-A-2 268 017 (CRC COMPAGNIA DI RICERCA CHIMICA S.A.)<br>----- | | |
| | | | **DOMAINES TECHNIQUES RECHERCHES (Int. Cl.5)** |
| | | | C 07 D 233/00 |

Le présent rapport a été établi pour toutes les revendications

| Lieu de la recherche | Date d'achèvement de la recherche | Examinateur |
|---|---|---|
| LA HAYE | 27-07-1990 | DE BUYSER I.A.F. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)